(19) **Europäisches Patentamt / European Patent Office / Office européen des brevets**

(11) **EP 4 084 681 B1**

(12) **EUROPEAN PATENT SPECIFICATION**

(45) Date of publication and mention
of the grant of the patent:
**03.09.2025 Bulletin 2025/36**

(21) Application number: **21715001.0**

(22) Date of filing: **19.02.2021**

(51) International Patent Classification (IPC):
**G16H 50/20** (2018.01)   **G16H 40/67** (2018.01)
**G16H 50/30** (2018.01)   **A61B 5/022** (2006.01)
**G16H 80/00** (2018.01)   **A61B 5/00** (2006.01)

(52) Cooperative Patent Classification (CPC):
**G16H 40/67; A61B 5/02241; A61B 5/7275;
A61B 5/746; G16H 50/20; G16H 50/30;
G16H 80/00;** G16H 20/40

(86) International application number:
**PCT/US2021/018827**

(87) International publication number:
**WO 2021/173445 (02.09.2021 Gazette 2021/35)**

(54) **HYPOTENSION PREDICTION WITH ADJUSTABLE HYPOTENSION THRESHOLD**

HYPOTONIEPROGNOSE MIT EINSTELLBARER HYPOTONIESCHWELLE

PRÉDICTION D'HYPOTENSION À SEUIL D'HYPOTENSION AJUSTABLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.02.2020 US 202062981179 P**

(43) Date of publication of application:
**09.11.2022 Bulletin 2022/45**

(73) Proprietor: **Becton, Dickinson and Company
Franklin Lakes, NJ 07417-1880 (US)**

(72) Inventors:
• **BUDDI, Sai, Prasad
Irvine, CA 92614 (US)**
• **SCHNEIDER, Brennan, Michael
Irvine, CA 92614 (US)**
• **JIAN, Zhongping
Irvine, CA 92614 (US)**
• **AL HATIB, Feras
Irvine, CA 92614 (US)**

(74) Representative: **Venner, Julia Ann et al
Kilburn & Strode LLP
Lacon London
84 Theobalds Road
London WC1X 8NL (GB)**

(56) References cited:
**US-A1- 2018 020 989**

• **SHIN SUNGTAE ET AL: "Forecasting
Hypotension during Vasopressor Infusion via
Time Series Analysis", 2019 41ST ANNUAL
INTERNATIONAL CONFERENCE OF THE IEEE
ENGINEERING IN MEDICINE AND BIOLOGY
SOCIETY (EMBC), IEEE, 23 July 2019
(2019-07-23), pages 498 - 501, XP033624991, DOI:
10.1109/EMBC.2019.8857084**

**Description**

BACKGROUND

The present disclosure relates generally to arterial blood pressure monitoring, and more specifically to prediction of hypotension with an adjustable hypotension threshold.

Hypotension, or low blood pressure, can be a harbinger of serious medical complications, and even mortality, for patients undergoing surgery and those acutely or critically ill patients receiving treatment in an intensive care unit (ICU). The dangers associated with the occurrence of hypotension in a patient are due both to the potential injury caused by the hypotension itself and to the many serious underlying medical disorders that the occurrence of hypotension may signify.

In and of itself, hypotension in surgical patients or critically ill patients is a serious medical condition. For example, in the operating room (OR) setting, hypotension during surgery is associated with increased mortality and organ injury. Even short durations of extreme hypotension during surgery are associated with acute kidney injury and myocardial injury. Among critically ill patients, in-hospital mortality may be nearly doubled for patients experiencing hypotension after emergency intubation. For surgical patients and seriously ill patients alike, hypotension, if not corrected, can impair organ perfusion, resulting in irreversible ischemic damage, neurological deficit, cardiomyopathy, and renal impairment.

In addition to posing serious risks to surgical patients and critically ill patients in its own right, hypotension can be a symptom of one or more other serious underlying medical conditions. Examples of underlying conditions for which hypotension may serve as an acute symptom include sepsis, myocardial infarction, cardiac arrhythmia, pulmonary embolism, hemorrhage, dehydration, anaphylaxis, acute reaction to medication, hypovolemia, insufficient cardiac output, and vasodilatory shock. Due to its association with such a variety of serious medical conditions, hypotension is relatively common, and is often seen as one of the first signs of patient deterioration in the OR and ICU.

Conventional patient monitoring for hypotension in the OR and ICU settings can include continuous or periodic blood pressure measurement. However, such monitoring, whether continuous or periodic, typically provides no more than a real-time assessment. As a result, hypotension in a surgical patient or critically ill patient is usually detected only after it begins to occur, so that remedial measures and interventions are not initiated until the patient has entered a hypotensive state. Although, as noted above, extreme hypotension can have potentially devastating medical consequences quite quickly, even relatively mild levels of hypotension can herald or precipitate cardiac arrest in patients with limited cardiac reserve.

In view of the frequency with which hypotension is observed to occur in the OR and ICU settings, and due to the serious and sometimes immediate medical consequences that can result when it does occur, a solution enabling prediction of a future hypotension event, before its occurrence, is highly desirable.

US 2018/0020989 describes systems and methods for performing mean arterial pressure (MAP) derived prediction of future hypotension. Such a system includes a hardware unit including a hardware processor and a system memory, a hypotension prediction software code stored in the system memory, and a sensory alarm. The hardware processor is configured to execute the hypotension prediction software code to receive MAP data of the living subject, and to transform the MAP data to one or more parameters predictive of a future hypotension event of the living subject. The hardware processor is further configured to execute the hypotension prediction software code to determine a risk score of the living subject corresponding to the probability of the future hypotension event based on at least some of the one or more parameters, and to invoke the sensory alarm if the risk score of the living subject satisfies a predetermined risk criteria.

Shin Sungtae et al "Forecasting Hypotension during Vasopressor Infusion via Time Series Analysis" (2019) 41st Annual International Conference of the IEEE Engineering in Medicine and Biology Society (EMBC), IEEE, 23 July 2019, pages 498-501, XP033624991, describes the investigation of two forecasting models, logistic regression (LR) and auto-regressive (AR) models, to predict sustained hypotension episodes (SHEs) in the ICU before the SHE occurred, for optimal management of hypotension during continuous vasopressor infusion.

SUMMARY

Aspects of the presently claimed invention are set out in the independent claims. Particular embodiments of these aspects are set out in the dependent claims. Any subject matter contained herein that does not fall within the scope of the appended claims is considered as being useful for understanding the invention.

In one example, a method for monitoring of arterial pressure of a patient and providing a warning to medical personnel of a predicted future hypotension event of the patient includes receiving, by a hemodynamic monitor, sensed hemodynamic data representative of an arterial pressure waveform of the patient. The method further includes offsetting, by the hemodynamic monitor, the received hemodynamic data based on a difference between a standard MAP threshold for hypotension and an adjusted MAP threshold for hypotension to produce adjusted hemodynamic data. The method further includes performing, by the hemodynamic monitor, waveform analysis of the adjusted hemodynamic data, determining, by the hemodynamic monitor based on the waveform analysis of the adjusted hemodynamic data, a risk score representing a probability of a future hypotension event for the patient, and invoking, by the hemodynamic monitor, a sensory alarm to produce a sensory signal in response to the risk score satisfying a predetermined risk criterion.

In another example, a system for monitoring of arterial pressure of a patient and providing a warning to medical personnel of a predicted future hypotension event includes a hemodynamic sensor, a system memory, a user interface, and a hardware processor. The hemodynamic sensor produces hemodynamic data representative of an arterial pressure waveform of the patient. The system memory stores hypotension prediction software code including a predictive weighting module. The user interface includes a sensory alarm that provides a sensory signal to warn the medical personnel of the predicted future hypotension event prior to the patient entering a hypotensive state. The hardware processor is configured to execute the hypotension prediction software code to offset the hemodynamic data representative of the arterial pressure waveform of the patient based on a difference between a standard mean arterial pressure (MAP) threshold for hypotension and an adjusted MAP threshold to produce adjusted hemodynamic data. The hardware processor is further configured to execute the hypotension prediction software code to perform waveform analysis of the adjusted hemodynamic data, and determine, using the predictive weighting module and based on the waveform analysis of the adjusted hemodynamic data, a risk score representing a probability of a future hypotension event for the patient. The hardware processor is further configured to execute the hypotension prediction software code to invoke the sensory alarm of the user interface in response to the risk score satisfying a predetermined risk criterion.

BRIEF DESCRIPTION OF THE DRAWINGS

FIG. 1 is a perspective view of an example hemodynamic monitor that determines a risk score representing a probability of a future hypotension event for a patient.

FIG. 2 is a perspective view of an example minimally invasive pressure sensor for sensing hemodynamic data representative of arterial pressure of a patient.

FIG. 3 is a perspective view of an example non-invasive sensor for sensing hemodynamic data representative of arterial pressure of a patient.

FIG. 4 is a block diagram illustrating an example hemodynamic monitoring system that determines a risk score representing a probability of a future hypotension event for a patient based on hemodynamic data that is adjusted based on a difference between a standard MAP threshold for hypotension and an adjusted MAP threshold.

FIG. 5A is a graph illustrating an example trace of an arterial pressure waveform including example indicia corresponding to the probability of future hypotension in a patient.

FIG. 5B is a graph illustrating an example trace of an adjusted arterial pressure waveform that is offset based on a difference between a standard MAP threshold for hypotension and an adjusted MAP threshold for hypotension.

FIG. 6 is a flow diagram illustrating example operations of the hemodynamic monitoring system to determine a risk score representing a probability of a future hypotension event using hemodynamic data that is adjusted based on a difference between a standard MAP threshold for hypotension and an adjusted MAP threshold for hypotension.

DETAILED DESCRIPTION

As described herein, a hemodynamic monitoring system implements a predictive risk model that produces a risk score representing a probability of a future hypotension event for a patient. The risk score is determined based on a weighted combination of a plurality of hypotension profiling parameters that are predictive of the future hypotension event. Risk coefficients that implement the weighting are selected based on a standard (or defined) mean arterial pressure (MAP) threshold for hypotension, such as a pressure of 65 millimeters of Mercury (mmHg) or other defined pressure threshold. The selection of risk coefficients and/or hypotension profiling parameters can be accomplished via training (e.g., offline training) of the predictive risk model using machine learning or other techniques to minimize a cost function representing the error of the predictive risk model output to the true value of training subsets that define hypotension according to the standard MAP threshold for hypotension.

According to techniques of this disclosure, the hemodynamic monitoring system can utilize an adjustable MAP threshold for hypotension to represent a modified hypotension pressure threshold. Rather than modify the predictive risk model (via retraining or otherwise) to accommodate the adjustable (e.g., user defined or otherwise adjusted) MAP threshold, the hemodynamic monitoring system adjusts the hemodynamic data representative of the sensed arterial pressure waveform of the patient. That is, rather than require retraining or other modification of the predictive risk model to determine new risk coefficients and/or hemodynamic profiling parameters based on the modified definition of hypotension (i.e., the adjusted MAP threshold), the hemodynamic monitoring system adjusts the input signal (i.e., the sensed hemodynamic data representative of the arterial pressure waveform of the patient) based on the difference between the standard MAP threshold and the adjusted MAP threshold. Waveform analysis is performed on the adjusted hemodynamic data to determine the risk score representing the probability of a future hypotension event.

Accordingly, a hemodynamic monitoring system implementing techniques of this disclosure can utilize an adjustable pressure threshold for hypotension without requiring retraining or other modifications to the predictive risk model, thereby enabling real-time updates to the hypotension threshold during operation in, e.g., an operating room (OR), an intensive care unit (ICU), or other patient care environment. As such, the system can provide a risk score representing a probability of future hypotension of the patient to enable timely and effective intervention while also taking advantage of the training and/or experience of medical personnel that could warrant the use of a modified hypotension threshold, thereby increasing usability of the system by the medical personnel for patient care.

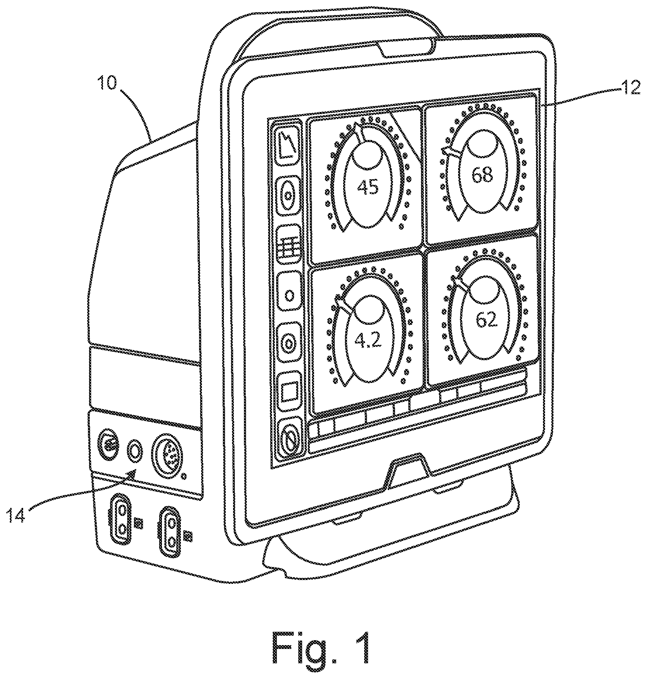

FIG. 1 is a perspective view of hemodynamic monitor 10 that determines a risk score representing a probability of a future hypotension event for a patient. As illustrated in FIG. 1, hemodynamic monitor 10 includes display 12 that, in the example of FIG. 1, presents a graphical user interface including control elements (e.g., graphical control elements) that enable user interaction with hemodynamic monitor 10. Hemodynamic monitor 10 can also include a plurality of input and/or output (I/O) connectors configured for wired connection (e.g., electrical and/or communicative connection) with one or more peripheral components, such as one or more hemodynamic sensors, as is further described below. For instance, as illustrated in FIG. 1, hemodynamic monitor 10 can include I/O connectors 14. While the example of FIG. 1 illustrates five separate I/O connectors 14, it should be understood that in other examples, hemodynamic monitor 10 can include fewer than five I/O connectors or greater than five I/O connectors. In yet other examples, hemodynamic monitor 10 may not include I/O connectors 14, but rather may communicate wirelessly with various peripheral devices.

As is further described below, hemodynamic monitor 10 includes one or more processors and computer-readable memory that stores hypotension prediction software code which is executable to produce a risk score representing a probability of a future hypotension event for a patient. For example, hemodynamic monitor 10 can receive sensed hemodynamic data representative of an arterial pressure waveform of the patient, such as via one or more hemodynamic sensors connected to hemodynamic monitor 10 via I/O connectors 14. Hemodynamic monitor 10 executes the hypotension prediction software code to obtain, using the received hemodynamic data, multiple hypotension profiling parameters, which can include one or more vital sign parameters characterizing vital sign data of the patient, as well as differential and combinatorial parameters derived from the one or more vital sign parameters, as is further described below. Hemodynamic monitor 10 further executes the hypotension prediction software code to apply a plurality of risk coefficients to the hypotension profiling parameters to produce a weighted combination resulting in the risk score that represents the probability of a future hypotension event for the patient. The plurality of risk coefficients, as described in further detail below, can be determined based on a standard mean arterial pressure (MAP) threshold, such as 65 mmHg, or other defined pressure threshold.

As described herein, hemodynamic monitor 10 can further utilize an adjusted MAP threshold for hypotension, the adjusted MAP threshold representing a deviation from the standard MAP threshold from which the coefficients utilized by the hypotension prediction software code are determined. For instance, hemodynamic monitor 10 can present graphical control elements (e.g., at a graphical user interface presented at display 12) that enable user input of an adjusted MAP threshold for hypotension, though inputs received via physical controls (e.g., buttons, knobs, or other physical input controls) are possible.

For example, as illustrated in FIG. 1, hemodynamic monitor 10 can present a graphical user interface at display 12. Display 12 can be a liquid crystal display (LCD), a light-emitting diode (LED) display, an organic light-emitting diode (OLED) display, or other display device suitable for providing information to users in graphical form. In some examples, such as the example of FIG. 1, display 12 can be a touch-sensitive and/or presence-sensitive display device configured to receive user input in the form of gestures, such as touch gestures, scroll gestures, zoom gestures, swipe gestures, or other gesture input. Hemodynamic monitor 10 presents control elements that enable user input of an adjusted MAP threshold, such as an absolute pressure (e.g., a MAP threshold value), a deviation value (e.g., a deviation from the standard MAP threshold), or other indication of an adjusted MAP threshold that can be, in some examples, user defined, such as by medical personnel.

Hemodynamic monitor 10, in response to receiving the adjusted MAP threshold, offsets the hemodynamic data representative of the arterial pressure waveform of the patient based on a difference between the standard MAP threshold for hypotension and the adjusted MAP threshold for hypotension to produce adjusted hemodynamic data, as is further described below. Hemodynamic monitor 10 executes the hypotension prediction software code to determine the risk score representing the probability of a future hypotension event for the patient using the risk coefficients that were determined based on the standard MAP threshold. Hemodynamic monitor 10 can invoke a sensory alarm, such as an audible alarm, a haptic alarm, or other sensory alarm in response to determining that the risk score satisfies predetermined risk criteria.

Accordingly, hemodynamic monitor 10 can provide a warning to medical personnel of a predicted future hypotension event of the patient prior to the patient entering a hypotensive state. Moreover, rather than require retraining or other modification of the hypotension prediction software code to determine new risk coefficients based on the adjusted MAP threshold, hemodynamic monitor 10 can determine the risk score using the risk coefficients that were determined based on the standard MAP threshold. As such, techniques of this disclosure can increase the usability of hemodynamic monitor 10 by enabling dynamic adaptation to an adjusted MAP threshold that may be based on training and expertise of medical personnel.

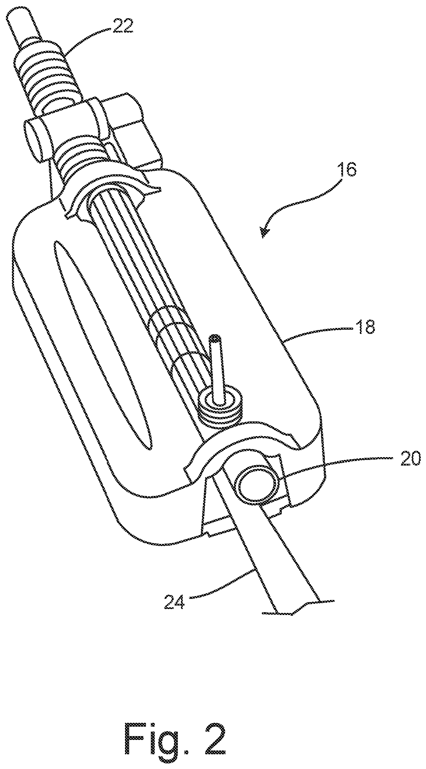

FIG. 2 is a perspective view of hemodynamic sensor 16 that can be attached to a patient for sensing hemodynamic data representative of arterial pressure of the patient. Hemodynamic sensor 16, illustrated in FIG. 2, is one example of a minimally invasive hemodynamic sensor that can be attached to the patient via, e.g., a radial arterial catheter inserted into an arm of the patient. In other examples, hemodynamic sensor 16 can attached to the patient via a femoral arterial catheter inserted into a leg of the patient.

As illustrated in FIG. 2, hemodynamic sensor 16 includes housing 18, fluid input port 20, catheter-side fluid port 22, and I/O cable 24. Fluid input port 20 is configured to be connected via tubing or other hydraulic connection to a fluid source, such as a saline bag or other fluid input source. Catheter-side fluid port 22 is configured to be connected via tubing or other hydraulic connection to a catheter (e.g., a radial arterial catheter or a femoral arterial catheter) that is inserted into an arm of the patient (i.e., a radial arterial catheter) or a leg of the patient (i.e., a femoral arterial catheter). I/O cable 24 is configured to connect to hemodynamic monitor 10 via, e.g., one or more of I/O connectors 14 (FIG. 1). Housing 18 of hemodynamic sensor 16 encloses one or more pressure transducers, communication circuitry, processing circuity, and corresponding electronic components to sense fluid pressure corresponding to arterial pressure of the patient that is transmitted to hemodynamic monitor 10 (FIG. 1) via I/O cable 24.

In operation, a column of fluid (e.g., saline solution) is introduced from a fluid source (e.g., a saline bag) through hemodynamic sensor 16 via fluid input port 20 to catheter-side fluid port 22 toward the catheter inserted into the patient. Arterial pressure is communicated through the fluid column to pressure sensors located within housing 16 which sense the pressure of the fluid column. Hemodynamic sensor 16 translates the sensed pressure of the fluid column to an electrical signal via the pressure transducers and outputs the corresponding electrical signal to hemodynamic monitor 10 (FIG. 1) via I/O cable 24. Hemodynamic sensor 16 therefore transmits analog sensor data (or a digital representation of the analog sensor data) to hemodynamic monitor 10 (FIG. 1) that is representative of substantially continuous beat-to-beat monitoring of the arterial pressure of the patient.

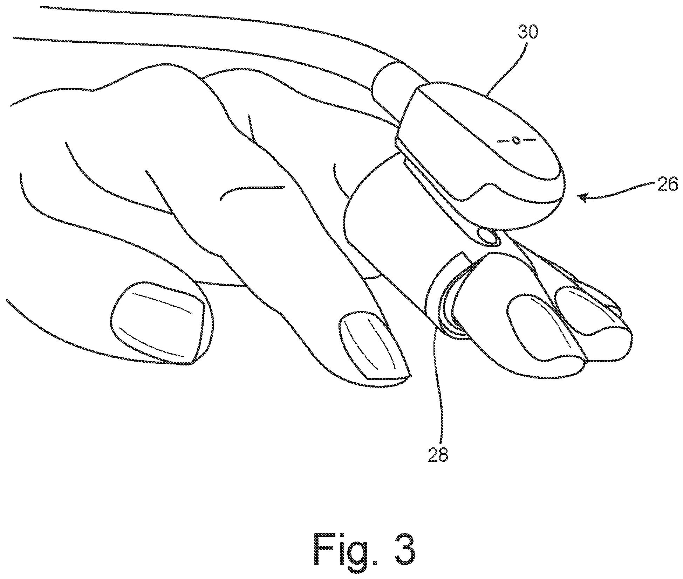

FIG. 3 is a perspective view of hemodynamic sensor 26 for sensing hemodynamic data representative of arterial pressure of a patient. Hemodynamic sensor 26, illustrated in FIG. 3, is one example of a non-invasive hemodynamic sensor that can be attached to the patient via one or more finger cuffs to sense data representative of arterial pressure of the patient. As illustrated in FIG. 3, hemodynamic sensor 26 includes inflatable finger cuff 28 and heart reference sensor 30. Inflatable finger cuff 28 includes an inflatable blood pressure bladder configured to inflate and deflate as controlled by a pressure controller (not illustrated) that is pneumatically connected to inflatable finger cuff 28. Inflatable finger cuff 28 also includes an optical (e.g., infrared) transmitter and an optical receiver that are electrically connected to heart reference sensor 30 to measure the changing volume of the arteries in the finger.

In operation, the pressure controller continually adjusts pressure within the finger cuff to maintain a constant volume of the arteries in the finger (i.e., the unloaded volume of the arteries) as measured by heart reference sensor 30 via the optical transmitter and optical receiver of inflatable finger cuff 28. The pressure applied by the pressure controller to continuously maintain the unloaded volume is representative of the blood pressure in the finger, and is communicated by the pressure controller to heart reference sensor 30. Heart reference sensor 30 translates the pressure signal representative of the blood pressure in the finger to hemodynamic data representative of the arterial pressure waveform of the patient, which is transmitted to hemodynamic monitor 10 (FIG. 1) via, e.g., I/O connectors 14 (FIG. 1). Accordingly, hemodynamic sensor 26 transmits sensor data that is representative of substantially continuous beat-to-beat monitoring of the arterial pressure of the patient.

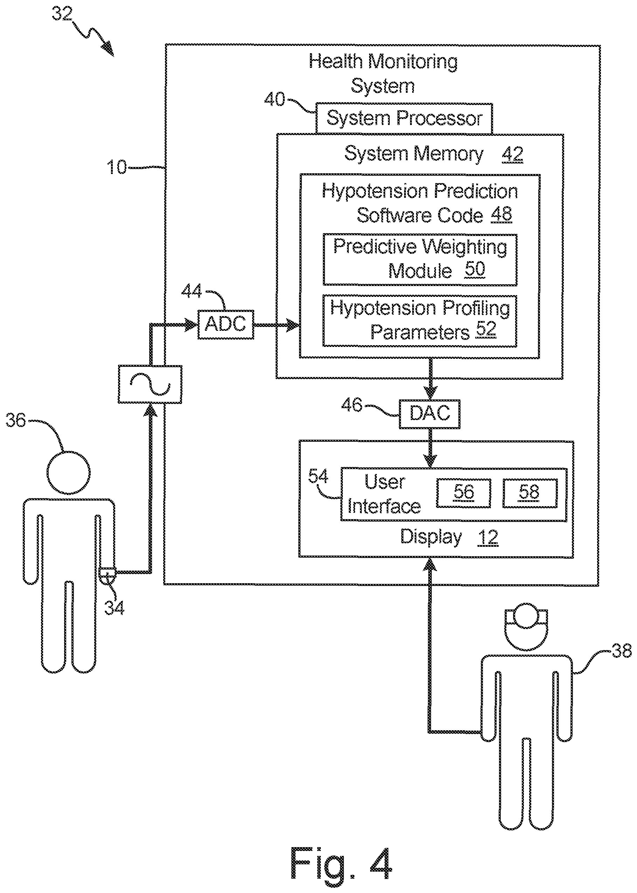

FIG. 4 is a block diagram of hemodynamic monitoring system 32 that determines a risk score representing a probability of a future hypotension event based on hemodynamic data that is adjusted based on a difference between a standard MAP threshold for hypotension and an adjusted MAP threshold. As illustrated in FIG. 4, hemodynamic monitoring system 32 includes hemodynamic monitor 10 and hemodynamic sensor 34. Hemodynamic monitoring system 32 can be implemented within a patient care environment, such as an ICU, an OR, or other patient care environment. As illustrated in FIG. 4, the patient care environment can include patient 36 and healthcare worker 38 trained to utilize hemodynamic monitoring system 32.

Hemodynamic monitor 10, as described above with respect to FIG. 1, can be, e.g., an integrated hardware unit including system processor 40, system memory 42, display 12, analog-to-digital (ADC) converter 44, and digital-to-analog (DAC) converter 46. In other examples, any one or more components and/or described functionality of hemodynamic monitor 10 can be distributed among multiple hardware units. For instance, in some examples, display 12 can be a separate display device that is remote from and operatively coupled with hemodynamic monitor 10. In general, though illustrated and described in the example of FIG. 4 as an integrated hardware unit, it should be understood that hemodynamic monitor 10 can include any combination of devices and components that are electrically, communicatively, or otherwise operatively connected to perform functionality attributed herein to hemodynamic monitor 10.

As illustrated in FIG. 4, system memory 42 stores hypotension prediction software code 48. Hypotension prediction software code 48 includes predictive weighting module 50 and hypotension profiling parameters 52. Display 12 provides user interface 54, which includes control elements 56 that enable user interaction with hemodynamic monitor 10 and/or other components of hemodynamic monitoring system 32. User interface 54, as illustrated in FIG. 4, also provides sensory alarm 58 to provide warning to medical personnel of a predicted future hypotension event of patient 36, as is further described below.

Hemodynamic sensor 34 can be attached to patient 36 to sense hemodynamic data representative of an arterial pressure waveform of patient 36. Hemodynamic sensor 34 is operatively connected to hemodynamic monitor 10 (e.g., electrically and/or communicatively connected via wired or wireless connection, or both) to provide the sensed hemodynamic data to hemodynamic monitor 10. In some examples, hemodynamic sensor 34 provides the hemodynamic data representative of the arterial pressure waveform of patient 36 to hemodynamic monitor 10 as an analog signal, which is converted by ADC 44 to digital hemodynamic data representative of the arterial pressure waveform. In other examples, hemodynamic sensor 34 can provide the sensed hemodynamic data to hemodynamic monitor 10 in digital form, in which case hemodynamic monitor 10 may not include or utilize ADC 44. In yet other examples, hemodynamic sensor 34 can provide the hemodynamic data representative of the arterial pressure waveform of patient 36 to hemodynamic monitor 10 as an analog signal, which is analyzed in its analog form by hemodynamic monitor 10.

Hemodynamic sensor 34 can be a non-invasive or minimally invasive sensor attached to patient 36. For instance, hemodynamic sensor 34 can take the form of minimally invasive hemodynamic sensor 16 (FIG. 2), non-invasive hemodynamic sensor 26 (FIG. 3), or other minimally invasive or non-invasive hemodynamic sensor. In some examples, hemodynamic sensor 34 can be attached non-invasively at an extremity of patient 36, such as a wrist, an arm, a finger, an ankle, a toe, or other extremity of patient 36. As such, hemodynamic sensor 34 can take the form of a small, lightweight, and comfortable hemodynamic sensor suitable for extended wear by patient 36 to provide substantially continuous beat-to-beat monitoring of the arterial pressure of patient 36 over an extended period of time, such as minutes or hours.

In certain examples, hemodynamic sensor 34 can be configured to sense an arterial pressure of patient 36 in a minimally invasive manner. For instance, hemodynamic sensor 34 can be attached to patient 36 via a radial arterial catheter inserted into an arm of patient 36. In other examples, hemodynamic sensor 34 can be attached to patient 36 via a femoral arterial catheter inserted into a leg of patient 36. Such minimally invasive techniques can similarly enable hemodynamic sensor 34 to provide substantially continuous beat-to-beat monitoring of the arterial pressure of patient 36 over an extended period of time, such as minutes or hours.

System processor 40 is configured to execute hypotension prediction software code 48, which implements predictive weighting module 50 utilizing hypotension profiling parameters 52 to produce a risk score representing a probability of a future hypotension event for patient 36. Examples of system processor 40 can include any one or more of a microprocessor, a controller, a digital signal processor (DSP), an application specific integrated circuit (ASIC), a field-programmable gate array (FPGA), or other equivalent discrete or integrated logic circuitry.

System memory 42 can be configured to store information within hemodynamic monitor 10 during operation. System memory 42, in some examples, is described as computer-readable storage media. In some examples, a computer-readable storage medium can include a non-transitory medium. The term "non-transitory" can indicate that the storage medium is not embodied in a carrier wave or a propagated signal. In certain examples, a non-transitory storage medium can store data that can, over time, change (e.g., in RAM or cache). System memory 42 can include volatile and non-volatile computer-readable memories. Examples of volatile memories can include random access memories (RAM), dynamic random access memories (DRAM), static random access memories (SRAM), and other forms of volatile memories. Examples of non-volatile memories can include, e.g., magnetic hard discs, optical discs, flash memories, or forms of electrically programmable memories (EPROM) or electrically erasable and programmable (EEPROM) memories.

Display 12 can be a liquid crystal display (LCD), a light-emitting diode (LED) display, an organic light-emitting diode (OLED) display, or other display device suitable for providing information to users in graphical form. User interface 54 can include graphical and/or physical control elements that enable user input to interact with hemodynamic monitor 10 and/or other components of hemodynamic monitoring system 32. In some examples, user interface 54 can take the form of a graphical user interface (GUI) that presents graphical control elements presented at, e.g., a touch-sensitive and/or presence sensitive display screen of display 12. In such examples, user input can be received in the form of gesture input, such as touch gestures, scroll gestures, zoom gestures, or other gesture input. In certain examples, user interface 54 can take the form of and/or include physical control elements, such as a physical buttons, keys, knobs, or other physical control elements configured to receive user input to interact with components of hemodynamic monitoring system 32.

In operation, hemodynamic sensor 34 senses hemodynamic data representative of an arterial pressure waveform of patient 36. Hemodynamic sensor 34 provides the hemodynamic data (e.g., as analog sensor data), to hemodynamic monitor 10. ADC 44 converts the analog hemodynamic data to digital hemodynamic data representative of the arterial pressure waveform of the patient.

System processor 40 executes hypotension prediction software code 48 to determine, using the received hemodynamic data, a risk score representing a probability of a future hypotension event for patient 36. For instance, system processor 40 can execute hypotension prediction software code 48 to obtain, using the received hemodynamic data, multiple hypotension profiling parameters 52. Hypotension profiling parameters 52 can include one or more vital sign parameters characterizing vital sign data of patient 36, as well as differential and combinatorial parameters derived from the one or more vital sign parameters, as is further described below.

Predictive weighting module 50 of hypotension prediction software code 48 determines a risk score corresponding to the probability of a future hypotension event for patient 36 based on a weighted combination of hypotension profiling parameters 52. That is, predictive weighting module 50 applies a plurality of risk coefficients stored at system memory 42 to hypotension profiling parameters 52 to produce the weighted combination resulting in the risk score. The risk coefficients can be determined via training operations (e.g., offline training) using machine learning or other techniques to minimize a cost function that represents the error of the risk score to the true value of training subsets (e.g., aggregations of data from multiple patients) that define hypotension according to a standard MAP threshold for hypotension. That is, risk coefficients utilized by predictive weighting module 50 can be selected via training operations to minimize the error of the predictive risk score determined by hypotension prediction software code 48 as predictive of a future hypotension event. The error of the predictive risk score to predict future hypotension events can be evaluated with respect to positive and negative training data subsets that define the occurrence of hypotension with respect to a standard (e.g., defined) MAP threshold, such as 65 mmHg or other pressure thresholds.

As described herein, hemodynamic monitor 10 can receive an adjusted MAP threshold for hypotension, such as a user defined MAP threshold via control elements 56 of user interface 54. The adjusted MAP threshold can represent a deviation from the standard MAP threshold by which risk coefficients utilized by predictive weighting module 50 are determined. The adjusted MAP threshold provided by, e.g., healthcare worker 38, can take the form of an absolute pressure (e.g., a MAP threshold value), a deviation value (e.g., a deviation from the standard MAP threshold), or other indication of an adjusted MAP threshold.

Hypotension prediction software code 48, in response to receiving the adjusted MAP threshold, offsets the hemodynamic data representative of the arterial pressure waveform of patient 36 based on a difference between the standard MAP threshold for hypotension and the adjusted MAP threshold, which can be received, e.g., via user interface 54 to produce adjusted digital hemodynamic data. For example, hypotension prediction software code 48 can add a difference between the standard MAP threshold and the adjusted MAP threshold to the received hemodynamic data representative of the arterial pressure waveform of patient 36. System processor 40 executes hypotension prediction software code 48 to determine hypotension profiling parameters 52 based on the adjusted hemodynamic data, and to determine the predictive risk score as the weighted combination of hypotension profiling parameters 52 using the risk coefficients that were determined based on the standard MAP threshold.

System processor 40 executes hypotension prediction software code 48 to invoke sensory alarm 58 via user interface 54 in response to determining that the risk score satisfies predetermined risk criteria, as is further described below. For instance, hypotension prediction software code 48 can invoke sensory alarm 58 to warn of a hypotension event predicted to occur, e.g., one to five minutes in the future, or up to approximately thirty minutes in the future. Sensory alarm 58 can be implemented as one or more of a visual alarm, an audible alarm, a haptic alarm, or other type of sensory alarm. For instance, sensory alarm 58 can be invoked as any combination of flashing and/or colored graphics shown by use interface 54 on display 12, display of the risk score via user interface 54 on display 12, a warning sound such as a siren or repeated tone, and a haptic alarm configured to cause hemodynamic monitor 10 to vibrate or otherwise deliver a physical impulse perceptible to healthcare worker 38 or other user.

Accordingly, hemodynamic monitor 10 provides a warning to medical personnel of a predicted future hypotension event of patient 36, thereby enabling timely and effective intervention to prevent the predicted future hypotension event. Moreover, rather than require retraining of the predictive risk model to determine new risk coefficients based on an adjusted MAP threshold, hemodynamic monitor 10, implementing techniques of this disclosure, offsets the sensed hemodynamic data received from hemodynamic sensor 34 based on a difference between the standard MAP threshold and the adjusted MAP threshold. Hemodynamic monitor 10 utilizes the adjusted hemodynamic data to determine the risk score using the unmodified risk coefficients, thereby enabling real-time updates by medical personnel to the MAP threshold defining hypotension. Techniques described herein therefore increase the usability of hemodynamic monitor 10 by enabling hemodynamic monitor 10 to adapt to, e.g., user defined changes that may be based on training and expertise of attending medical personnel to predict a future hypotension event for patient 36.

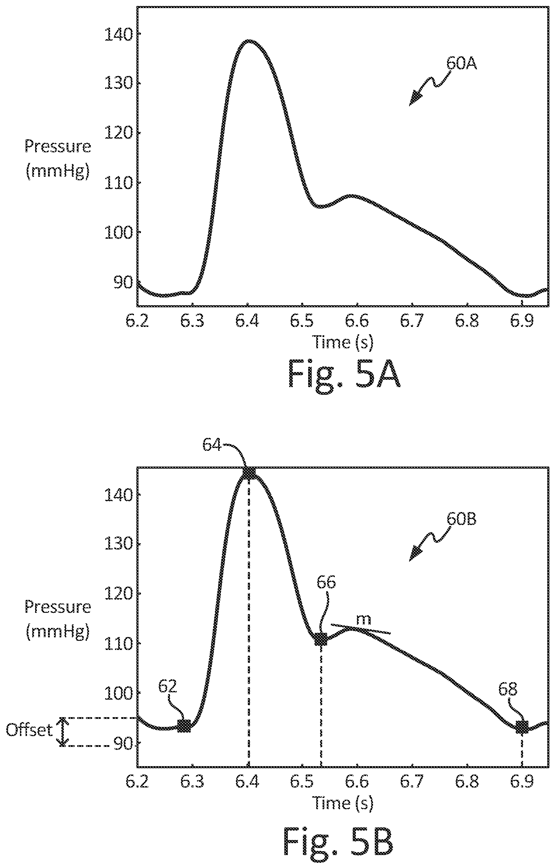

FIGS. 5A and 5B are graphs illustrating example traces of arterial pressure waveforms prior to offset (FIG. 5A) and subsequent to an applied offset (FIG. 5B), the offset being determined based on a difference between a standard MAP threshold for hypotension and an adjusted MAP threshold for hypotension. For purposes of clarity and ease of discussion, FIGS. 5A and 5B are described below together and with reference to hemodynamic sensing system 32 of FIG. 4.

FIG. 5A is a graph illustrating an example trace of arterial pressure waveform 60A corresponding to hemodynamic data sensed by hemodynamic sensor 34 and received by hemodynamic monitor 10. FIG. 5B is a graph illustrating an example trace of arterial pressure waveform 60B representing arterial pressure waveform 60A after application of an offset by hypotension prediction software code 48 based on a difference between a standard MAP threshold for hypotension and an adjusted MAP threshold for hypotension.

As illustrated in FIGS. 5A and 5B, in this example, hypotension prediction software code 48 applies an offset of 5 mmHg to arterial pressure waveform 60A to produce adjusted arterial pressure waveform 60B. In the example of FIGS. 5A and 5B, the applied offset of 5 mmHg corresponds to a difference of 5 mmHg between the standard MAP threshold for hypotension and the adjusted MAP threshold for hypotension. That is, in the example of FIGS. 5A and 5B, an adjusted MAP threshold for hypotension is provided, e.g., via control elements 56 of user interface 54. System processor 40 executes hypotension prediction software code 48 to determine a difference between the standard MAP threshold (e.g., 65 mmHg) and the adjusted MAP threshold (e.g., 60 mmHg) which, in this example, corresponds to a difference of 5 mmHg, though other differences are possible (e.g., more than 5 mmHg or less than 5 mmHg). Hypotension prediction software code 48, executed by system processor 40, applies the offset to hemodynamic waveform 60A to produce adjusted hemodynamic waveform 60B representing waveform 60A having an offset of 5 mmHg added consistently throughout hemodynamic waveform 60A.

While the example of FIGS. 5A and 5B are described with respect to a positive offset (i.e., an offset of 5 mmHg) applied to hemodynamic waveform 60A to produce adjusted hemodynamic waveform 60B, it should be understood that negative offsets are also possible. For example, if an adjusted MAP threshold for hypotension is provided (e.g., by a user) that is greater than the standard MAP threshold for hypotension, the applied offset is negative. Conversely, as described with respect to the example of FIGS. 5A and 5B, if an adjusted MAP threshold for hypotension is provided (e.g., by a user) that is less than the standard MAP threshold for hypotension, the applied offset is positive.

System processor 40 executes hypotension prediction software code 48 to determine hypotension profiling parameters 52 based on adjusted arterial pressure waveform 60B. Predictive weighting module 50 applies risk coefficients determined based on the standard MAP threshold (e.g., 65 mmHg) to determine the risk score representing a probability of a future hypotension event for patient 36.

As further illustrated in FIG. 5B, adjusted hemodynamic waveform 60B (e.g., represented via digital hemodynamic data) can include various indicia predictive of a future hypotension event for patient 36. FIG. 5B illustrates example indicia 62, 64, 66, and 68, corresponding respectively to the start of a heart beat (indicium 62), the maximum systolic pressure marking the end of systolic rise (indicium 64), the presence of the dicrotic notch marking the end of systolic decay (indicium 66), and the diastole of the heartbeat (indicium 68) of patient 36. Also shown in FIG. 5B is example slope "m" of adjusted arterial pressure waveform 60B, though it should be understood that slope "m" is merely representative of multiple slopes that may be determined at multiple locations along adjusted arterial pressure waveform 60B.

Additional indicia predictive of future hypotension for patient 36 can be extracted from adjusted hemodynamic waveform 60B by hypotension prediction software code 48 based on behavior of adjusted hemodynamic waveform 60B in various intervals, such as in the interval from the maximum systolic pressure at indicium 64 to the diastole at indicium 66, as well as the interval from the start of the heartbeat at indicium 62 to the diastole at indicium 66. The behavior of adjusted arterial pressure waveform 60B during intervals: 1) systolic rise 62-64, 2) systolic decay 64-66, 3) systolic phase 62-66, 4) diastolic phase 66-68, 5) interval 64-68, and 6) heartbeat interval 62-68, can be determined by hypotension prediction software code 48 by determining the area under the curve of adjusted hemodynamic waveform 60B and the standard deviation of adjusted hemodynamic waveform 60B in each of intervals 1-6. The respective areas and standard deviations determined for intervals 1-6 can serve as additional indicia predictive of future hypotension for patient 36.

Accordingly, hemodynamic monitor 10 implementing techniques of this disclosure can offset the received hemodynamic data sensed by hemodynamic sensor 34 based on a difference between a standard MAP threshold for hypotension and an adjusted MAP threshold for hypotension to produce adjusted hemodynamic data. Rather than retrain or otherwise modify the predictive model implemented by hypotension prediction software code 48 to determine modified risk coefficients based on the adjusted MAP threshold, hemodynamic monitor 10 can offset the input signal (i.e., hemodynamic data received from hemodynamic sensor 34) for use with the unmodified risk coefficients that are determined based on the standard MAP threshold for hypotension. As such, hemodynamic monitor 10 can determine a risk score representing a probability of a future hypotension event for patient 36 utilizing the unmodified risk coefficients while accommodating the user defined MAP threshold for hypotension.

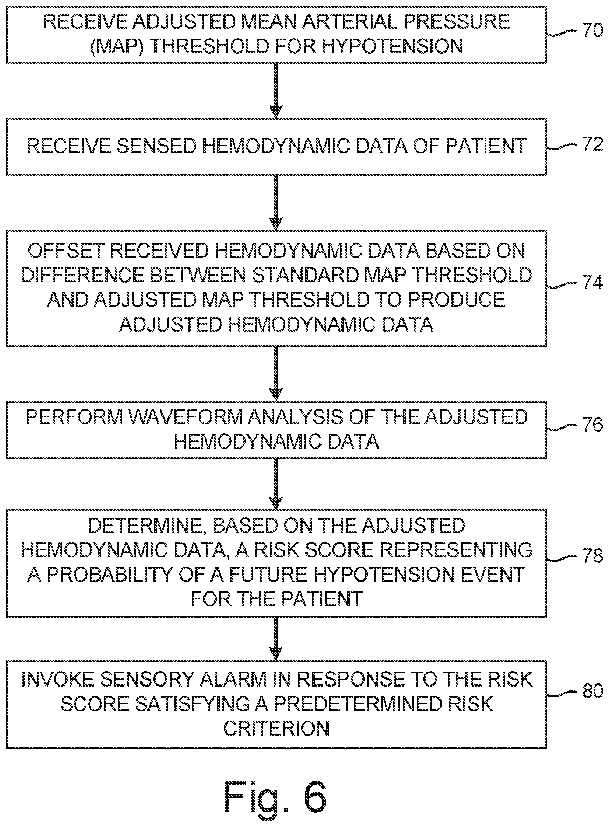

FIG. 6 is a flow diagram illustrating example operations to determine a risk score representing a probability of a future hypotension event using hemodynamic data that is adjusted based on a difference between a standard MAP threshold for hypotension and an adjusted MAP threshold for hypotension. For purposes of clarity and ease of discussion, the example operations are described below within the context of hemodynamic monitoring system 32 of FIG. 4.

An adjusted MAP threshold for hypotension is received by hemodynamic monitor 10 (Step 70). For example, hemodynamic monitor 10 can receive an adjusted MAP threshold for hypotension provided by, e.g., healthcare worker 38 via control elements 56 of user interface 54. Hemodynamic monitor 10 receives sensed hemodynamic data representative of an arterial pressure waveform of patient 36 (Step 72). For instance, hemodynamic monitor 10 can receive an analog hemodynamic sensor signal representative of an arterial pressure waveform of patient 36 from hemodynamic sensor 34.

Hemodynamic monitor 10 offsets the received hemodynamic data based on a difference between a standard MAP threshold for hypotension and the adjusted MAP threshold for hypotension to produce adjusted hemodynamic data (Step 74). For example, system processor 40 can execute hypotension prediction software code 48 to determine a difference between the standard MAP threshold and the adjusted MAP threshold. Hypotension prediction software code 48 can offset hemodynamic data received from hemodynamic sensor 34 by adding the difference between the standard MAP threshold for hypotension and the adjusted MAP threshold to the received hemodynamic data. In some examples, hypotension prediction software code 48 can add the difference between the standard MAP threshold for hypotension and the adjusted MAP threshold for hypotension according to the following equation:

$$f'(t) = f(t) + (\theta - \theta') \qquad \text{(Equation 1)}$$

where $f'(t)$ is the adjusted hemodynamic data, $f(t)$ is the received hemodynamic data, $\theta$ is the standard MAP threshold for hypotension, and $\theta'$ is the adjusted MAP threshold for hypotension.

System processor 40 of hemodynamic monitor 10 executes hypotension prediction software code 48 to perform waveform analysis of the adjusted hemodynamic data (Step 76). For example, system processor 40 can execute hypotension prediction software code 48 to perform waveform analysis of the adjusted hemodynamic data to obtain hypotension profiling parameters 52 that are predictive of future hypotension in patient 36. Hypotension profiling parameters 52 can include one or more of vital sign parameters characterizing vital sign data of patient 36, differential parameters derived from the vital sign parameters, and combinatorial parameters representing combinations of one or more of the vital sign parameters and differential parameters.

Vital sign parameters characterizing vital sign data can include, e.g., stroke volume, heart rate, respiration, cardiac contractility, mean arterial pressure, baroreflex sensitivity measures, hemodynamic complexity measures, frequency domain hemodynamic features, or other vital sign parameters. Baroreflex sensitivity measures quantify the relationship between complementary physiological processes. For example, a decrease in blood pressure in a healthy patient is typically compensated by an increase in heart rate and/or an increase in peripheral resistance. The baroreflex sensitivity measures that may be included in the one or more vital sign parameters characterizing vital sign data correspond to the degree to which patient 36 is responding appropriately to normal physiological variations.

Hemodynamic complexity measures quantify the amount of regularity in cardiac measurements over time, as well as the entropy, e.g., the unpredictability of fluctuations in cardiac measurements over time. For instance, unpredictable cardiac fluctuations are a normal phenomenon associated with health. Very low entropy, i.e., a high degree of regularity in cardiac measurements over time and the substantial absence of unpredictable fluctuations, can be a significant warning sign of an impending hypotension event. Frequency domain hemodynamic features quantify various measures of cardiac performance as a function of frequency rather than time.

Hypotension predication software code 48 can further determine differential parameters based on the one or more vital sign parameters characterizing vital sign data of patient 36. Hypotension prediction software code 48 can derive the differential parameters from the one or more vital sign parameters by determining variations of the one or more vital sign parameters with respect to time, with respect to frequency, or with respect to other parameters from among the one or more vital sign parameters. As a result, each of one or more vital sign parameters can give rise to one, two, or several differential parameters included among hypotension profiling parameters 52.

For example, the differential parameter stroke volume variation (SVV) can be derived based on changes in the parameter stroke volume (SV) as a function of time and/or as a function of sampling frequency. Similarly, changes in mean arterial pressure (ΔMAP) can be derived as a differential parameter with respect to time and/or sampling frequency. As a further example, changes in MAP with respect to time can be derived by subtracting the average of the MAP over the past five minutes, ten minutes, or other time durations, from the current value of the MAP.

Hypotension prediction software code 48 can generate combinatorial parameters included in hypotension profiling parameters 52 using the one or more vital sign parameters and the derived differential parameters. For example, the combinatorial parameters can be generated using the one or more vital sign parameters and the differential parameters by generating a power combination of a subset of the one or more vital sign parameters and the differential parameters. For instance, each of the combinatorial parameters can be generated as a power combination of three parameters, which can be randomly or purposefully selected, from among the one or more vital sign parameters characterizing vital sign data and/or the differential parameters. Each of the three parameters selected from among the one or more vital sign parameters and/or the differential parameters can be raised to an exponential power, and can be multiplied with or added to the other two parameters analogously raised to an exponential power. The exponential power to which each of the three parameters selected from the one or more vital sign parameters and/or the differential parameters is raised can be, but need not be, the same exponential power.

In some examples, generation of the combinatorial parameters can be performed using a predetermined and limited integer range of exponential powers. For instance, the exponential powers used to generate the combinatorial parameters can be integer powers selected from among negative two, negative one, zero, one, and two (-2, -1, 0, 1, 2). As such, each combinatorial parameter can be expressed, in some examples, according to the following equation:

$$X = Y_1^a * Y_2^b * \ldots Y_n^c \qquad \text{(Equation 2)}$$

where $Y$ is one of one or more vital sign parameters characterizing vital sign data or one of the differential parameters, $n$ is any integer greater than two, and each of $a$, $b$, and $c$ can be any one of -2, -1, 0, 1, and 2. In some examples, Equation 2 above can be applied to substantially all possible power combinations of the one or more vital sign parameters, the differential parameters, and the one or more vital sign parameters with the differential parameters, subject to the predetermined constraints described above (i.e., the value of $n$ being any integer greater than two, and each of $a$, $b$, and $c$ being selected from the group consisting of -2, -1, 0, 1, and 2).

Hypotension profiling parameters 52 include one or more vital sign parameters characterizing vital sign data, the differential parameters, and the combinatorial parameters. As such, hypotension prediction software code 48 determines hypotension profiling parameters 52 by identifying one or more vital sign parameters characterizing vital sign data based on the adjusted hemodynamic data, obtaining the differential parameters based on one or more vital sign parameters, and generating the combinatorial parameters using one or more of vital sign parameters and the differential parameters.

A risk score representing a probability of a future hypotension event for patient 36 is determined based on the waveform analysis of the adjusted hemodynamic data (Step 78). For example, system processor 40 can execute hypotension prediction software code 48 to cause predictive weighting module 50 to determine the risk score as a weighted combination of hypotension profiling parameters 52. Predictive weighting module 50 can determine the weighted combination of hypotension profiling parameters 52 by applying a plurality of risk coefficients to hypotension profiling parameters 52, which include the vital sign parameters characterizing vital sign data of patient 36, the differential parameters derived from the vital sign parameters, and the combinatorial parameters. The plurality of risk coefficients applied by predictive weighting module 50 can be determined (e.g., via offline training) with respect to the standard MAP threshold for hypotension. As such, because hypotension prediction software code 48 determines the risk score based on hypotension profiling parameters 52, which are derived from hemodynamic data sensed by hemodynamic sensor attached to patient 36, it should be noted that hypotension prediction software code 48 determines the risk score for patient 36 without direct comparison to hypotension in other patients and without direct reference to a hypotension database that may store information regarding hypotension in patients other than patient 36.

In some examples, predictive weighting module 50 determines the risk score representing the probability of a future hypotension event for patient 36 according to the following equation:

$$R = 1/(1 + e^{-A}) \qquad \text{(Equation 3)}$$

where $R$ is the risk score, and $A$ is expressed as:

$$\begin{aligned}
A = {} & c_0 + c_1 \times v_1 + c_2 \times v_2 + c_3 \times v_3 + c_4 \times v_4 + c_5 \times v_5 + c_6 \times v_6 + c_7 \times v_7^2 \times v_8^2 \times v_9^{-2} \\
& + c_8 \times v_2^2 \times v_{10} \times v_{11}^{-1} + c_9 \times \Delta(v_{12}^2 \times v_{13}^2 \times v_{14}) + c_{10} \times \Delta(v_{15}^2 \times v_1 \times v_{16}^{-1}) \\
& + c_{11} \times \Delta(v_{17}^2 \times v_{18}^2 \times v_{19}^{-2})
\end{aligned}$$

where:

$v_1$ = CWI, the cardiac work indexed by the body surface area of patient 36;
$v_2$ = MAPavg, the averaged mean arterial pressure;
$v_3$ = $\Delta$MAPavg, the change of the averaged mean arterial pressure MAPavg when compared to an initial state;
$v_4$ = avgSysDec, the averaged pressure at the decay portion of the systolic phase;
$v_5$ = $\Delta$Sys, the change of systolic pressure when compared to initial values;
$v_6$ = ppAreaNor, the normalized area under the adjusted arterial pressure waveform;
$v_7$ = biasDia, the bias of the diastolic slope;
$v_8$ = CW, the cardiac work;
$v_9$ = mapDnlocArea, the area under the adjusted arterial pressure waveform, between first instance of MAP and the dicrotic notch;
$v_{10}$ = SWcomb, the stroke work;
$v_{11}$ = ppArea, the area under the adjusted arterial pressure waveform;
$v_{12}$ = decAreaNor, the normalized area of the decay phase;
$v_{13}$ = slopeSys, the slope of the systolic phase;
$v_{14}$ = Cwk, the Windkessel compliance;
$v_{15}$ = sys_rise_area_nor, the normalized area under the systolic rise phase;
$v_{16}$ = pulsepres, the pulse pressure;

$v_{17}$ = avg_sys, the averaged pressure of the systolic phase;

$v_{18}$ = dpdt2, the maximum value of the second order derivative of the adjusted arterial pressure waveform;

$v_{19}$ = dpdt, the maximum value of the first order derivative of the adjusted arterial pressure waveform; and

$c_0, c_1, ..., c_{11}$ are the risk coefficients determined with respect to the standard MAP threshold for hypotension.

In some examples, the risk score $R$ can be expressed as a fraction, as represented by Equation 3 above. In other examples, the risk score can be converted to a percentage risk score between zero percent and one hundred percent.

Hemodynamic monitor 10 invokes a sensory alarm in response to the risk score satisfying a predetermined risk criterion (Step 80). For instance, hypotension prediction software code 48 can invoke sensory alarm 58 of user interface 54 in response to determining that the risk score R determined according to Equation 3 above satisfies a predetermined risk criterion. In some examples, the output of hypotension prediction software code 48 can be processed using DAC 46 to convert digital signals into analog signals for presentation via user interface 54 at display 12.

The predetermined risk criterion can be based on the value of the risk score, on the trend of the risk score over a time interval, or both. For instance, where the risk score is expressed as a percentage between zero and one hundred, hypotension prediction software code 48 can invoke sensory alarm 58 (e.g., immediately) in response to determining that the risk score exceeds a first predetermined threshold, such as eighty-five percent. In some examples, hypotension prediction software code 48 can invoke sensory alarm 58 in response to determining that the risk score satisfies a second predetermined threshold over the entirety of a first predetermined time period. In such examples, the second predetermined threshold can be lower than the first predetermined threshold.

As such, hypotension prediction software code 48 can invoke sensory alarm 58, e.g., immediately, in response to determining that the risk score exceeds the first predetermined threshold (e.g., eighty-five percent). Hypotension prediction software code 48 can also invoke sensory alarm 58 in response to determining that the risk score exceeds a second predetermined threshold (e.g., eighty percent) that is less than the first predetermined threshold for the first predetermined time period (e.g., ten to thirty seconds) during which the risk score is continuously greater than the second predetermined threshold (eighty percent) and less than the first predetermined threshold (e.g., eighty five percent). In certain examples, hypotension prediction software code 48 can invoke sensory alarm 58 in response to determining that the risk score is greater than a third predetermined threshold that is less than the second predetermined threshold for a second predetermined time period (e.g., one or more minutes). In yet other examples, hypotension prediction software code 48 can invoke sensory alarm 58 in response to determining that the risk score exceeds a fourth predetermined threshold (e.g., seventy-five percent) a threshold number of times (e.g., two times, three times, or other numbers of times) over a third predetermined time period (e.g., one minute, two minutes, or other time periods).

Although not illustrated in the example operations of FIG. 6, in some examples, hemodynamic monitor 10 can identify, using hypotension prediction software code 48, a most probable cause of a predicted future hypotension event of patient 36. For example, based on identified indicia, hypotension prediction software code 48 can identify poor vascular tone, low blood volume, reduced cardiac contractility, or other most probable causes of a predicted future hypotension event of patient 36.

In some examples, hemodynamic monitor 10 can recommend a medical intervention for preventing the predicted future hypotension event of patient 36, such as by identifying a recommended medical intervention corresponding to the identified most probable cause of the predicted future hypotension event for patient 36. For instance, with respect to a most probable cause of poor vascular tone, hemodynamic monitor 10 can recommend a medical intervention of administration of a vasoconstrictor. With respect to a most probable cause of low blood volume, for example, hemodynamic monitor 10 can recommend a medical intervention of administration of saline or whole blood.

Accordingly, hemodynamic monitor 10 implementing techniques of this disclosure provides a risk score predictive of a future hypotension event for patient 36, thereby enabling timely and effective intervention to prevent the hypotension event prior to patient 36 entering a hypotensive state. Moreover, by enabling adjustment to a defined hypotension threshold without requiring retraining of the predictive risk model, the techniques described herein increase usability of hemodynamic monitoring system 32 to accommodate, e.g., the training and experience of medical personnel.

While the invention has been described with reference to an exemplary embodiment(s), it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiment(s) disclosed, but that the invention will include all embodiments falling within the scope of the appended claims.

## Claims

1.   A method for monitoring of arterial pressure of a patient and providing a warning to medical personnel of a predicted future hypotension event of the patient, the method comprising:

receiving (72), by a hemodynamic monitor, sensed hemodynamic data representative of an arterial pressure waveform of the patient; offsetting (74), by the hemodynamic monitor, the received hemodynamic data based on a difference between a standard mean arterial pressure (MAP) threshold for hypotension and an adjusted MAP threshold for hypotension that is different than the standard MAP threshold for hypotension to produce adjusted hemodynamic data, wherein offsetting the received hemodynamic data comprises adding the difference between the standard MAP threshold for hypotension and the adjusted MAP threshold to the received hemodynamic data;

performing (76), by the hemodynamic monitor, waveform analysis of the adjusted hemodynamic data to determine a plurality of hypotension profiling parameters predictive of the future hypotension event for the patient; determining (78), by the hemodynamic monitor based on the waveform analysis of the adjusted hemodynamic data, a risk score representing a probability of a future hypotension event for the patient, wherein determining the risk score representing the probability of the future hypotension event for the patient comprises applying a plurality of risk coefficients to the plurality of hypotension profiling parameters to determine the risk score, wherein the plurality of risk coefficients are determined based on the standard MAP threshold; and

invoking (80), by the hemodynamic monitor, a sensory alarm to produce a sensory signal in response to the risk score satisfying a predetermined risk criterion.

2. The method of claim 1,
wherein adding the difference between the standard MAP threshold for hypotension and the adjusted MAP threshold to the received hemodynamic data comprises adding the difference between the standard MAP threshold for hypotension and the adjusted MAP threshold to the received hemodynamic data according to the following equation:

$$f'(t) = f(t) + (\theta - \theta')$$

wherein $f'(t)$ is the adjusted hemodynamic data;
wherein $f(t)$ is the received hemodynamic data;
wherein $\theta$ is the standard MAP threshold for hypotension; and
wherein $\theta'$ is the adjusted MAP threshold for hypotension.

3. The method of claim 1 or claim 2,
wherein performing the waveform analysis of the adjusted hemodynamic data to determine the plurality of hypotension profiling parameters predictive of the future hypotension event for the patient comprises:

performing the waveform analysis of the adjusted hemodynamic data to obtain vital sign parameters from the adjusted hemodynamic data;
deriving differential parameters based on one or more of the vital sign parameters; and
generating combinatorial parameters using one or more of the vital sign parameters and/or one or more of the differential parameters;
wherein the plurality of hypotension profiling parameters include one or more of the vital sign parameters, the differential parameters, and the combinatorial parameters.

4. The method of claim 3,
wherein the vital sign parameters include one or more of stroke volume, heart rate, respiration, and cardiac contractibility.

5. The method of claim 3 or claim 4,
wherein deriving the differential parameters based on one or more of the vital sign parameters comprises deriving the differential parameters to represent variations in the one or more of the vital sign parameters with respect to time, with respect to frequency, or with respect to other vital sign parameters.

6. The method of any of claims 3 to 5,
wherein generating the combinatorial parameters comprises generating the combinatorial parameters as a combination of vital sign parameters, a combination of differential parameters, or a combination of at least one vital sign parameter and at least one differential parameter.

7. The method of any preceding claim, further comprising:
receiving, by the hemodynamic monitor, the adjusted MAP threshold for hypotension via a user interface of the hemodynamic monitor.

8. A system (32) for monitoring of arterial pressure of a patient (36) and providing a warning to medical personnel (38) of a predicted future hypotension event, the system comprising:

a hemodynamic sensor (34) that produces hemodynamic data representative of an arterial pressure waveform of the patient;
a system memory (42) that stores hypotension prediction software code (48) including a predictive weighting module (50);
a user interface (54) that includes a sensory alarm (58) that provides a sensory signal to warn the medical personnel of the predicted future hypotension event prior to the patient entering a hypotensive state; and
a hardware processor (40) that is configured to execute the hypotension prediction software code to:

offset the hemodynamic data representative of the arterial pressure waveform of the patient based on a difference between a standard MAP threshold for hypotension and an adjusted MAP threshold to produce adjusted hemodynamic data, wherein the hardware processor is configured to execute the hypotension prediction software code to offset the hemodynamic data by adding the difference between the standard MAP threshold for hypotension and the adjusted MAP threshold to the hemodynamic data;
perform waveform analysis of the adjusted hemodynamic data by determining a plurality of hypotension profiling parameters (52) predictive of the future hypotension event for the patient;
determine, using the predictive weighting module and based on the waveform analysis of the adjusted hemodynamic data, a risk score representing a probability of a future hypotension event for the patient, wherein the hardware processor is configured to execute the hypotension prediction software code to determine the risk score representing the probability of the future hypotension event for the patient by applying, using the predictive weighting module, a plurality of risk coefficients to the plurality of hypotension profiling parameters to determine the risk score, wherein the plurality of risk coefficients are determined based on the standard MAP threshold; and
invoke the sensory alarm of the user interface in response to the risk score satisfying a predetermined risk criterion.

9. The system of claim 8,
wherein the hardware processor is configured to execute the hypotension prediction software code to add the difference between the standard MAP threshold for hypotension and the adjusted MAP threshold to the hemodynamic data according to the following equation:

$$f'(t) = f(t) + (\theta - \theta')$$

wherein $f'(t)$ is the adjusted hemodynamic data;
wherein $f(t)$ is the hemodynamic data;
wherein $\theta$ is the standard MAP threshold for hypotension; and
wherein $\theta'$ is the adjusted MAP threshold for hypotension.

10. The system of claim 8 or claim 9,
wherein the hardware processor is configured to execute the hypotension prediction software code to determine the plurality of hypotension profiling parameters predictive of the future hypotension event for the patient by executing the hypotension prediction software code to:

perform the waveform analysis of the adjusted hemodynamic data to obtain vital sign parameters from the adjusted hemodynamic data;
derive differential parameters based on one or more of the vital sign parameters; and
generate combinatorial parameters using one or more of the vital sign parameters and/or one or more of the differential parameters;
wherein the plurality of hypotension profiling parameters include one or more of the vital sign parameters, the differential parameters, and the combinatorial parameters.

**11.** The system of claim 10,
wherein the vital sign parameters include one or more of stroke volume, heart rate, respiration, and cardiac contractibility.

**12.** The system of claim 10 or claim 11,
wherein the hardware processor is configured to execute the hypotension prediction software code to derive the differential parameters based on one or more of the vital sign parameters by deriving the differential parameters to represent variations in the one or more of the vital sign parameters with respect to time, with respect to frequency, or with respect to other vital sign parameters.

**13.** The system of any of claims 10 to 12,
wherein the hardware processor is configured to execute the hypotension prediction software code to generate the combinatorial parameters by generating the combinatorial parameters as a combination of vital sign parameters, a combination of differential parameters, or a combination of at least one vital sign parameter and at least one differential parameter.

**14.** The system of any of claims 8 to 13, wherein the hemodynamic sensor (34):

a) is a noninvasive hemodynamic sensor (26) that is attachable to an extremity of the patient; or
b) is a minimally invasive arterial catheter based hemodynamic sensor (16); or
c) produces the hemodynamic data as an analog hemodynamic sensor signal representative of the arterial pressure waveform of the patient, the system optionally
further comprising: an analog-to-digital converter (44) that converts the analog hemodynamic sensor signal to digital hemodynamic data representative of the arterial pressure waveform of the patient.

**15.** The system of any of claims 8 to 14,
wherein the user interface (54) further includes control elements (56) that enable user input of the adjusted MAP threshold for hypotension.

**Patentansprüche**

**1.** Verfahren zum Überwachen des arteriellen Drucks eines Patienten und zum Bereitstellen einer Warnung für medizinisches Personal vor einem vorhergesagten zukünftigen Hypotonieereignis des Patienten, wobei das Verfahren umfasst:

Empfangen (72), durch eine hämodynamische Überwachungsvorrichtung, von erfassten hämodynamischen Daten, die eine arterielle Druckwellenform des Patienten darstellen;
Verrechnen (74), durch die hämodynamische Überwachungsvorrichtung, der empfangenen hämodynamischen Daten basierend auf einer Differenz zwischen einem Standardschwellenwert für den mittleren arteriellen Druck (MAP, Mean Arterial Pressure) für Hypotonie und einem angepassten MAP-Schwellenwert für Hypotonie, der von dem Standard-MAP-Schwellenwert für Hypotonie verschieden ist, um angepasste hämodynamische Daten zu erzeugen, wobei das Verrechnen der empfangenen hämodynamischen Daten umfasst, die Differenz zwischen dem Standard-MAP-Schwellenwert für Hypotonie und dem angepassten MAP-Schwellenwert zu den empfangenen hämodynamischen Daten zu addieren;
Durchführen (76), durch die hämodynamische Überwachungsvorrichtung, einer Wellenformanalyse der angepassten hämodynamischen Daten, um mehrere Hypotonie-Profilierungsparameter zu bestimmen, die das zukünftige Hypotonieereignis für den Patienten vorhersagen;
Bestimmen (78), durch die hämodynamische Überwachungsvorrichtung basierend auf der Wellenformanalyse der angepassten hämodynamischen Daten, eines Risikowertes, der eine Wahrscheinlichkeit eines zukünftigen Hypotonieereignisses für den Patienten darstellt, wobei das Bestimmen des Risikowertes, der die Wahrscheinlichkeit des zukünftigen Hypotonieereignisses für den Patienten darstellt, umfasst, mehrere Risikokoeffizienten auf die mehreren Hypotonie-Profilierungsparametern anzuwenden, um den Risikowert zu bestimmen, wobei die mehreren Risikokoeffizienten basierend auf dem Standard-MAP-Schwellenwert bestimmt werden; und
Veranlassen (80), durch die hämodynamische Überwachungsvorrichtung, eines sensorischen Alarms, um in Reaktion darauf, dass der Risikowert ein vorbestimmtes Risikokriterium erfüllt, ein sensorisches Signal zu erzeugen.

**2.** Verfahren nach Anspruch 1,
wobei das Addieren der Differenz zwischen dem Standard-MAP-Schwellenwert für Hypotonie und dem angepassten MAP-Schwellenwert zu den empfangenen hämodynamischen Daten umfasst, die Differenz zwischen dem Standard-MAP-Schwellenwert für Hypotonie und dem angepassten MAP-Schwellenwert zu den empfangenen hämodynamischen Daten zu addieren, gemäß der folgenden Gleichung:

$$f'(t) = f(t) + (\theta - \theta')$$

wobei f'(t) für die angepassten hämodynamischen Daten steht;
wobei f(t) für die empfangenen hämodynamischen Daten steht;
wobei $\theta$ für den Standard-MAP-Schwellenwert für Hypotonie steht; und
wobei $\theta'$ für den angepassten MAP-Schwellenwert für Hypotonie steht.

**3.** Verfahren nach Anspruch 1 oder Anspruch 2,
wobei das Durchführen der Wellenformanalyse der angepassten hämodynamischen Daten, um die mehreren Hypotonie-Profilierungsparameter zu bestimmen, die das zukünftige Hypotonieereignis für den Patienten vorhersagen, umfasst:

Durchführen der Wellenformanalyse der angepassten hämodynamischen Daten, um Vitalparameter aus den angepassten hämodynamischen Daten zu erhalten;
Ableiten von Differenzialparametern basierend auf einem oder mehreren der Vitalparameter; und
Erzeugen kombinatorischer Parameter unter Verwendung eines oder mehrerer Vitalparameter und/oder eines oder mehrerer Differentialparameter;
wobei die mehreren Hypotonie-Profilierungsparameter einen oder mehrere der Vitalparameter, Differenzialparameter und kombinatorischen Parameter beinhalten.

**4.** Verfahren nach Anspruch 3,
wobei die Vitalparameter einen oder mehrere der Parameter Schlagvolumen, Herzfrequenz, Atmung und Kontraktilität des Herzens umfassen.

**5.** Verfahren nach Anspruch 3 oder Anspruch 4,
wobei das Ableiten der Differenzialparameter basierend auf einem oder mehreren der Vitalparameter umfasst, die Differenzialparameter abzuleiten, um Variationen in den ein oder mehreren Vitalparametern in Bezug auf die Zeit, in Bezug auf die Frequenz oder in Bezug auf andere Vitalparameter darzustellen.

**6.** Verfahren nach einem der Ansprüche 3 bis 5,
wobei das Erzeugen der kombinatorischen Parameter umfasst, die kombinatorischen Parameter als eine Kombination von Vitalparametern, eine Kombination von Differenzialparametern oder eine Kombination von mindestens einem Vitalparameter und mindestens einem Differenzialparameter zu erzeugen.

**7.** Verfahren nach einem der vorstehenden Ansprüche, ferner umfassend:
Empfangen, durch die hämodynamische Überwachungsvorrichtung, des angepassten MAP-Schwellenwertes für Hypotonie über eine Benutzerschnittstelle der hämodynamischen Überwachungsvorrichtung.

**8.** System (32) zum Überwachen des arteriellen Drucks eines Patienten (36) und zum Bereitstellen einer Warnung für medizinisches Personal (38) vor einem vorhergesagten zukünftigen Hypotonieereignis, wobei das System umfasst:

einen hämodynamischen Sensor (34), der hämodynamische Daten erzeugt, die eine arterielle Druckwellenform des Patienten darstellen;
einen Systemspeicher (42), der Hypotonieprognose-Softwarecode (48) speichert und ein Prognose-Gewichtungsmodul (50) aufweist;
eine Benutzerschnittstelle (54), die einen sensorischen Alarm (58) aufweist, der ein sensorisches Signal bereitstellt, um das medizinische Personal vor dem vorhergesagten zukünftigen Hypotonieereignis zu warnen, bevor der Patient in einen hypotensiven Zustand gerät; und
einen Hardwareprozessor (40), der dafür ausgelegt ist, den Hypotonieprognose-Softwarecode auszuführen zum:

Verrechnen der hämodynamischen Daten, die die arterielle Druckwellenform des Patienten darstellen, basierend auf einer Differenz zwischen einem Standard-MAP-Schwellenwert für Hypotonie und einem angepassten MAP-Schwellenwert, um angepasste hämodynamische Daten zu erzeugen, wobei der Hardwareprozessor dafür ausgelegt ist, den Hypotonieprognose-Softwarecode auszuführen, um die hämodynamischen Daten zu verrechnen, indem die Differenz zwischen dem Standard-MAP-Schwellenwert für Hypotonie und dem angepassten MAP-Schwellenwert zu den hämodynamischen Daten addiert wird;

Durchführen der Wellenformanalyse der angepassten hämodynamischen Daten durch Bestimmen der mehreren Hypotonie-Profilierungsparameter (52), die das zukünftige Hypotonieereignis für den Patienten vorhersagen;

Bestimmen, unter Verwendung des Prognose-Gewichtungsmoduls und basierend auf der Wellenformanalyse der angepassten hämodynamischen Daten, eines Risikowertes, der eine Wahrscheinlichkeit eines zukünftigen Hypotonieereignisses für den Patienten darstellt, wobei der Hardwareprozessor ausgelegt ist zum Ausführen des Hypotonieprognose-Softwarecodes, um den Risikowert zu bestimmen, der die Wahrscheinlichkeit des zukünftigen Hypotonieereignisses für den Patienten darstellt, indem unter Verwendung des Prognose-Gewichtungsmoduls mehrere Risikokoeffizienten auf die mehreren Hypotonie-Profilierungsparameter angewendet werden, um den Risikowert zu bestimmen, wobei die mehreren Risikokoeffizienten basierend auf dem Standard-MAP-Schwellenwert bestimmt werden; und

Veranlassen des sensorischen Alarms der Benutzerschnittstelle in Reaktion darauf, dass der Risikowert ein vorbestimmtes Risikokriterium erfüllt.

9. System nach Anspruch 8,
wobei der Hardwareprozessor dafür ausgelegt ist, den Hypotonieprognose-Softwarecode auszuführen, um die Differenz zwischen dem Standard-MAP-Schwellenwert für Hypotonie und dem angepassten MAP-Schwellenwert zu den hämodynamischen Daten zu addieren, gemäß der folgenden Gleichung:

$$f'(t) = f(t) + (\theta - \theta')$$

wobei f'(t) für die angepassten hämodynamischen Daten steht;
wobei f(t) für die hämodynamischen Daten steht;
wobei $\theta$ für den Standard-MAP-Schwellenwert für Hypotonie steht; und
wobei $\theta'$ für den angepassten MAP-Schwellenwert für Hypotonie steht.

10. System nach Anspruch 8 oder Anspruch 9,
wobei der Hardwareprozessor dafür ausgelegt ist, den Hypotonieprognose-Softwarecode auszuführen, um die mehreren Hypotonie-Profilierungsparameter zu bestimmen, die das zukünftige Hypotonieereignis für den Patienten vorhersagen, indem der Hypotonieprognose-Softwarecode ausgeführt wird zum:

Durchführen der Wellenformanalyse der angepassten hämodynamischen Daten, um Vitalparameter aus den angepassten hämodynamischen Daten zu erhalten;
Ableiten von Differenzialparametern basierend auf einem oder mehreren der Vitalparameter; und
Erzeugen kombinatorischer Parameter unter Verwendung eines oder mehrerer Vitalparameter und/oder eines oder mehrerer Differentialparameter;
wobei die mehreren Hypotonie-Profilierungsparameter einen oder mehrere der Vitalparameter, Differenzialparameter und kombinatorischen Parameter beinhalten.

11. System nach Anspruch 10,
wobei die Vitalparameter einen oder mehrere der Parameter Schlagvolumen, Herzfrequenz, Atmung und Kontraktilität des Herzens umfassen.

12. System nach Anspruch 10 oder Anspruch 11,
wobei der Hardwareprozessor ausgelegt ist zum Ausführen des Hypotonieprognose-Softwarecodes zum Ableiten der Differenzialparameter basierend auf einem oder mehreren der Vitalparameter, indem die Differenzialparameter abgeleitet werden, um Variationen in den ein oder mehreren Vitalparametern in Bezug auf die Zeit, in Bezug auf die Frequenz oder in Bezug auf andere Vitalparameter darzustellen.

13. System nach einem der Ansprüche 10 bis 12,
wobei der Hardwareprozessor ausgelegt ist zum Ausführen des Hypotonieprognose-Softwarecodes zum Erzeugen der kombinatorischen Parameter, indem die kombinatorischen Parameter als eine Kombination von Vitalparametern, eine Kombination von Differenzialparametern oder eine Kombination von mindestens einem Vitalparameter und mindestens einem Differenzialparameter abgeleitet werden.

**14.** System nach einem der Ansprüche 8 bis 13,
wobei der hämodynamische Sensor (34):

a) ein nichtinvasiver hämodynamischer Sensor (26) ist, der an einer Extremität des Patienten angebracht werden kann; oder

b) ein minimal invasiver hämodynamischer Sensor (16) auf Basis eines Arterienkatheters ist; oder

c) die hämodynamischen Daten als ein analoges hämodynamisches Sensorsignal erzeugt, das die arterielle Druckwellenform des Patienten darstellt, wobei das System optional ferner umfasst: einen Analog-Digital-Wandler (44), der das analoge hämodynamische Sensorsignal in digitale hämodynamische Daten umwandelt, die die arterielle Druckwellenform des Patienten darstellen.

**15.** System nach einem der Ansprüche 8 bis 14,
wobei die Benutzerschnittstelle (54) ferner Bedienelemente (56) aufweist, die eine Benutzereingabe des angepassten MAP-Schwellenwertes für Hypotonie ermöglichen.

## Revendications

**1.** Procédé de surveillance de la pression artérielle d'un patient et d'avertissement du personnel médical à propos d'un événement d'hypotension futur prédit du patient, le procédé comprenant :

la réception (72), par un moniteur hémodynamique, de données hémodynamiques détectées représentatives d'une forme d'onde de pression artérielle du patient ;

le décalage (74), par le moniteur hémodynamique, des données hémodynamiques reçues sur la base d'une différence entre un seuil standard de pression artérielle moyenne (MAP) pour l'hypotension et un seuil ajusté de MAP pour l'hypotension qui est différent du seuil standard de MAP pour l'hypotension pour produire des données hémodynamiques ajustées, dans lequel le décalage des données hémodynamiques reçues comprend l'ajout de la différence entre le seuil standard de MAP pour l'hypotension et le seuil ajusté de MAP aux données hémodynamiques reçues ;

la réalisation (76), par le moniteur hémodynamique, d'une analyse de forme d'onde des données hémodynamiques ajustées pour déterminer une pluralité de paramètres de profilage d'hypotension prédictifs de l'événement d'hypotension futur pour le patient ;

la détermination (78), par le moniteur hémodynamique sur la base de l'analyse de forme d'onde des données hémodynamiques ajustées, d'un score de risque représentant la probabilité d'un événement d'hypotension futur pour le patient, dans lequel la détermination du score de risque représentant la probabilité de l'événement d'hypotension futur pour le patient comprend l'application d'une pluralité de coefficients de risque à la pluralité de paramètres de profilage d'hypotension pour déterminer le score de risque, dans lequel la pluralité de coefficients de risques est déterminée sur la base du seuil de MAP standard ; et

l'invocation (80), par le moniteur hémodynamique, d'une alarme sensorielle pour produire un signal sensoriel en réponse au fait que le score de risque satisfait à un critère de risque prédéfini.

**2.** Procédé selon la revendication 1,
dans lequel l'ajout de la différence entre le seuil de MAP standard pour l'hypotension et le seuil de MAP ajusté aux données hémodynamiques reçues comprend l'ajout de la différence entre le seuil de MAP standard pour l'hypotension et le seuil de MAP ajusté aux données hémodynamiques reçues selon l'équation suivante :

$$f'(t) = f(t) + (\theta - \theta')$$

où f' (t) représente les données hémodynamiques ajustées ;
où f(t) représente les données hémodynamiques reçues ;
où $\theta$ est le seuil de MAP standard pour l'hypotension ; et
où $\theta'$ est le seuil de MAP ajusté pour l'hypotension.

**3.** Procédé selon la revendication 1 ou la revendication 2,
dans lequel la réalisation de l'analyse de forme d'onde des données hémodynamiques ajustées pour déterminer la pluralité de paramètres de profilage d'hypotension prédictifs de l'événement d'hypotension futur pour le patient comprend :

la réalisation de l'analyse de forme d'onde des données hémodynamiques ajustées pour obtenir des paramètres de signes vitaux à partir des données hémodynamiques ajustées ;
la dérivation de paramètres différentiels sur la base d'un ou de plusieurs des paramètres de signes vitaux ; et
la génération de paramètres combinatoires à l'aide d'un ou de plusieurs des paramètres de signes vitaux et/ou d'un ou de plusieurs des paramètres différentiels ;
dans lequel la pluralité de paramètres de profilage d'hypotension comporte un ou plusieurs des paramètres de signes vitaux, des paramètres différentiels, et des paramètres combinatoires.

**4.** Procédé selon la revendication 3,
dans lequel les paramètres de signes vitaux comportent un ou plusieurs parmi volume systolique, fréquence cardiaque, respiration, et contractibilité cardiaque.

**5.** Procédé selon la revendication 3 ou la revendication 4,
dans lequel la dérivation des paramètres différentiels sur la base d'un ou de plusieurs des paramètres de signes vitaux consiste à dériver les paramètres différentiels pour représenter des variations du ou des paramètres de signes vitaux par rapport au temps, par rapport à la fréquence, ou par rapport à d'autres paramètres de signes vitaux.

**6.** Procédé selon l'une quelconque des revendications 3 à 5,
dans lequel la génération des paramètres combinatoires comprend la génération des paramètres combinatoires en tant que combinaison de paramètres de signes vitaux, combinaison de paramètres différentiels, ou combinaison d'au moins un paramètre de signes vitaux et d'au moins un paramètre différentiel.

**7.** Procédé selon une quelconque revendication précédente, comprenant en outre :
la réception, par le moniteur hémodynamique, du seuil de MAP ajusté pour l'hypotension par le biais d'une interface utilisateur du moniteur hémodynamique.

**8.** Système (32) de surveillance de la pression artérielle d'un patient (36) et d'avertissement du personnel médical (38) à propos d'un événement d'hypotension futur prédit du patient, le système comprenant :

un capteur hémodynamique (34) qui produit des données hémodynamiques représentatives d'une forme d'onde de pression artérielle du patient ;
une mémoire système (42) qui stocke un code logiciel de prédiction d'hypotension (48) comportant un module de pondération prédictive (50) ;
une interface utilisateur (54) qui comporte une alarme sensorielle (58) qui fournit un signal sensoriel pour avertir le personnel médical de l'événement d'hypotension futur prédit avant que le patient entre dans un état d'hypotension ; et
un processeur matériel (40) qui est configuré pour exécuter le code logiciel de prédiction d'hypotension pour :

décaler les données hémodynamiques représentatives de la forme d'onde de pression artérielle du patient en fonction d'une différence entre un seuil de MAP standard pour l'hypotension et un seuil de MAP ajusté pour produire des données hémodynamiques ajustées, dans lequel le processeur matériel est configuré pour exécuter le code logiciel de prédiction d'hypotension pour décaler les données hémodynamiques en ajoutant la différence entre le seuil de MAP standard pour l'hypotension et le seuil de MAP ajusté aux données hémodynamiques ;
la réalisation d'une analyse de forme d'onde des données hémodynamiques ajustées en déterminant une pluralité de paramètres de profilage d'hypotension (52) prédictifs de l'événement d'hypotension futur pour le patient ;
la détermination, à l'aide du module de pondération prédictive et sur la base de l'analyse de forme d'onde des données hémodynamiques ajustées, d'un score de risque représentant une probabilité d'un événement d'hypotension futur pour le patient, dans lequel le processeur matériel est configuré pour exécuter le code logiciel de prédiction d'hypotension pour déterminer le score de risque représentant la probabilité d'un événement d'hypotension futur pour le patient en appliquant, au moyen du module de pondération prédictive, une pluralité de coefficients de risque à la pluralité de paramètres de profilage d'hypotension pour déterminer le score de risque, dans lequel la pluralité de coefficients de risque est déterminée sur la base du seuil de MAP standard ; et

l'invocation de l'alarme sensorielle de l'interface utilisateur en réponse au fait que le score de risque satisfait à un critère de risque prédéfini.

**9.** Système selon la revendication 8,
dans lequel le processeur matériel est configuré pour exécuter le code logiciel de prédiction d'hypotension pour ajouter la différence entre le seuil de MAP standard pour l'hypotension et le seuil de MAP ajusté aux données hémodynamiques selon l'équation suivante :

$$f'(t) = f(t) + (\theta - \theta')$$

où f' (t) représente les données hémodynamiques ajustées ;
où f(t) représente les données hémodynamiques ;
où $\theta$ est le seuil de MAP standard pour l'hypotension ; et
où $\theta'$ est le seuil de MAP ajusté pour l'hypotension.

**10.** Système selon la revendication 8 ou la revendication 9,
dans lequel le processeur matériel est configuré pour exécuter le code logiciel de prédiction d'hypotension pour déterminer la pluralité de paramètres de profilage d'hypotension prédictifs de l'événement d'hypotension futur pour le patient en exécutant le code logiciel de prédiction d'hypotension pour :

réaliser l'analyse de forme d'onde des données hémodynamiques ajustées pour obtenir des paramètres de signes vitaux à partir des données hémodynamiques ajustées ;
dériver des paramètres différentiels sur la base d'un ou de plusieurs des paramètres de signes vitaux ; et
générer des paramètres combinatoires à l'aide d'un ou de plusieurs des paramètres de signes vitaux et/ou d'un ou de plusieurs des paramètres différentiels ;
dans lequel la pluralité de paramètres de profilage d'hypotension comporte un ou plusieurs des paramètres de signes vitaux, des paramètres différentiels, et des paramètres combinatoires.

**11.** Système selon la revendication 10,
dans lequel les paramètres de signes vitaux comportent un ou plusieurs parmi volume systolique, fréquence cardiaque, respiration, et contractibilité cardiaque.

**12.** Système selon la revendication 10 ou la revendication 11,
dans lequel le processeur matériel est configuré pour exécuter le code logiciel de prédiction d'hypotension pour dériver les paramètres différentiels sur la base d'un ou de plusieurs des paramètres de signes vitaux en dérivant les paramètres différentiels pour représenter des variations du ou des paramètres de signes vitaux par rapport au temps, par rapport à la fréquence, ou par rapport à d'autres paramètres de signes vitaux.

**13.** Système selon l'une quelconque des revendications 10 à 12,
dans lequel le processeur matériel est configuré pour exécuter le code logiciel de prédiction d'hypotension pour générer les paramètres combinatoires en générant les paramètres combinatoires sous la forme d'une combinaison de paramètres de signes vitaux, d'une combinaison de paramètres différentiels, ou d'une combinaison d'au moins un paramètre de signes vitaux et d'au moins un paramètre différentiel.

**14.** Système selon l'une quelconque des revendications 8 à 13,
dans lequel le capteur hémodynamique (34) :

a) est un capteur hémodynamique non invasif (26) qui peut être fixé à une extrémité du patient ; ou
b) est un capteur hémodynamique à base de cathéter artériel minimalement invasif (16) ; ou
c) produit les données hémodynamiques sous la forme d'un signal de capteur hémodynamique analogique représentatif de la forme d'onde de pression artérielle du patient, le système comprenant éventuellement en outre : un convertisseur analogique-numérique (44) qui convertit le signal de capteur hémodynamique analogique en données hémodynamiques numériques représentatives de la forme d'onde de pression artérielle du patient.

**15.** Système selon l'une quelconque des revendications 8 à 14,
dans lequel l'interface utilisateur (54) comporte en outre des éléments de commande (56) qui permettent à l'utilisateur d'entrer le seuil de MAP ajusté pour l'hypotension.

Fig. 1

Fig. 2

Fig. 3

Fig. 4

Fig. 5A

Fig. 5B

RECEIVE ADJUSTED MEAN ARTERIAL PRESSURE (MAP) THRESHOLD FOR HYPOTENSION — 70

RECEIVE SENSED HEMODYNAMIC DATA OF PATIENT — 72

OFFSET RECEIVED HEMODYNAMIC DATA BASED ON DIFFERENCE BETWEEN STANDARD MAP THRESHOLD AND ADJUSTED MAP THRESHOLD TO PRODUCE ADJUSTED HEMODYNAMIC DATA — 74

PERFORM WAVEFORM ANALYSIS OF THE ADJUSTED HEMODYNAMIC DATA — 76

DETERMINE, BASED ON THE ADJUSTED HEMODYNAMIC DATA, A RISK SCORE REPRESENTING A PROBABILITY OF A FUTURE HYPOTENSION EVENT FOR THE PATIENT — 78

INVOKE SENSORY ALARM IN RESPONSE TO THE RISK SCORE SATISFYING A PREDETERMINED RISK CRITERION — 80

## Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20180020989 A

**Non-patent literature cited in the description**

- Forecasting Hypotension during Vasopressor Infusion via Time Series Analysis. **SHIN SUNGTAE et al.** 41st Annual International Conference of the IEEE Engineering in Medicine and Biology Society (EMBC). IEEE, 23 July 2019, 498-501